# EUROPEAN PATENT APPLICATION

(11) **EP 2 875 821 A1**
(43) Date of publication of application: **27.05.2015**
(21) Application number: 14380011.8
(22) Date of filing: 11.03.2014
(51) Int. Cl.: A61K 36/28

(54) **Natural liquid composition against infections**

(30) Priority: 26.11.2013 ES 201331717 P
(71) Applicant: Perez, Federico Luiz, Andorra La Vella (AD)
(72) Inventor: Perez, Federico Luiz, Andorra La Vella (AD)
(74) Representative: Espiell Volart, Eduardo Maria

(57) **Abstract**

This liquid composition comprises between 0.5 and 1 millilitre of 70° ethyl alcohol for every 10 millilitres of an infusion of chamomile (Chamomilla recutita or Matricaria Chamomilla), naturally grown, without chemical fertilisers or pesticides, prepared with 1 gram of chamomile for every 0.2 litres of low-mineralisation mineral water. For a specific treatment against psoriasis, this composition further includes organic sweet almond oil in a quantity ranging between 3 and 6 millilitres for every 10 millilitres of chamomile infusion.

## Description

### Object of the invention

The present invention relates to an addition to patent of invention No. 200400524 for "Natural liquid composition against infections", currently in full force and effect, said liquid composition being composed of all-natural substances, which include chamomile, mineral water and ethyl alcohol.

### Field of the invention

This invention has application within the industry dedicated to the manufacturing of pharmaceutical products, as well as the industry dedicated to the manufacturing of products obtained from natural and similar substances for topical use.

### Background of the invention

Currently, there are various liquid products prepared with natural plants in the market, which are used to treat any type of infection, the main characteristic of said liquids being the combination of different natural plants, to obtain the healing effect of said products through the mixture and combination of said natural plants.

Prior to the application for patent of invention no. 200400524, the applicant was not aware of the existence of any healing liquid against any type of infection with the combination of products considered in said invention, due to the fact that the latter is prepared solely by using a single natural plant, specifically chamomile (Chamomilla recutita or Matricaria chamomilla).

The natural liquid composition against infections disclosed in the applicant's aforementioned patent 200400524 is characterised by the fact that it is prepared with natural substances such as medicinal plants and natural mineral water from the Pyrenees, and by the combination of said products with 70° alcohol boricated to saturation, to obtain a liquid that, when applied on the affected areas, is not counterproductive for the nature of the skin.

The liquid composition of the aforementioned patent of invention 200400524 may be used advantageously to heal any type of infection that may appear in the human body.

Specifically, said liquid composition is obtained by the combination of different products, the main element being the use of a single natural plant, which is combined with Natural Water from the Pyrenees, to create an infusion that is combined with 70° Alcohol boricated to saturation, resulting in a liquid that is effective in heating any type of infection.

The products that make up the liquid proposed by the invention are the following. Namely:
- Chamomilla recutita or Matricaria Chamomilla, known as chamomile; thanks to its composition, said plant is particularly appropriate for protection of the skin against contaminant agents, whilst exhibiting carminative, spasmolytic, sedative, antiemetic, tonic, antiphlogistic, diaphoretic and stomachic activities that conjugate perfectly to disinfect any part of the human body. Ormenis essential oil is used as a calmative oil in both mental and digestive terms. It is especially recommended for sensitive skins. It has a long-lasting fresh, rich, balsamic, sweet aroma, and its essential properties include being an excellent muscle relaxant and skin conditioner; moreover, chamomile also has antimicrobial properties, and it is also known that it inhibits the growth of those bacteria known as staphylococci and streptococci. Furthermore, chamomile is an exceptionally safe plant; apart from the fact that a small number of people may present allergic reactions, chamomile does not cause negative effects in people.
- Natural Mineral Water from the Pyrenees, the water with the lowest salt content, the chemical composition whereof is 201.0 mg/l of Dry Residue, 74.4 mg/l of calcium, 0.6 mg/l of Sodium, 1.4 mg/l of magnesium, 207.0 mg/l of bicarbonates, 13.9 mg/l of Sulfates, 1.0 mg/l of chlorides.
- 70° Alcohol boricated to saturation, composed of a solution of 70 degrees alcohol, distilled water and saturated boric acid, which presents bacteriostatic and antifungal properties and is used to clean any area infected by an ailment, due to the fact that boricated Alcohol creates an unfavourable medium for bacterial proliferation; the proportion of the elements composing said product is the following for 100 ml thereof: 70 ml of ethyl Alcohol, 30 ml of distilled water and 5% of boric acid.

In said patent of invention no. 200400524, the aforementioned products are combined with one another in a proportion of 10 millilitres of infusion of Chamomilla recutita or Matricaria Chamomilla and natural mineral water from the Pyrenees per 1 millilitre of 70° Alcohol Boricated to Saturation; the infusion is made by combining 1 gram of Chamomilla recutita or Matricaria Chamomilla and 0.2 litres of natural mineral water from the Pyrenees. Once these products have been combined, they are allowed to macerate for approximately 1 hour, and are subsequently combined with 70° Alcohol boricated to saturation in the specified proportion of 10 ml of the aforementioned infusion per 1 ml of 70° alcohol boricated to saturation, to obtain the liquid of the present invention.

Whilst fulfilling the specified objectives and applications, the Liquid composition of the aforementioned patent of invention no. 200400524 presents certain disadvantages, primarily due to the incorporation of 70° alcohol boricated to saturation.

70° alcohol boricated to saturation is very difficult to produce on an industrial scale in large quantities and has a high cost, for which reason the liquid composition is excessively expensive for commercialisation.

Another disadvantage of this liquid composition is that, in order to preserve large quantities thereof, it requires special care due precisely to its composition.

Currently, 70° alcohol boricated to saturation is prepared in small quantities in pharmacies, by means of extemporaneous preparations, and it is prescribed for the treatment of otitis. However, its use for a prolonged period of time is prohibited, since it may cause hearing losses. Moreover, its use produces very unpleasant stinging sensations in the auditory canal, and it is not indicated in its pure form for the treatment of infections in human beings. The presence of boric acid in the liquid composition of patent of invention no. 200400524 prevents said composition from being used for the treatment of otitis in children under 3 years of age. When it is used for the treatment of otitis and the liquid dries in the ear, the boric acid contained in said composition produces a micrometer-sized dust layer that may cause hearing loss in older people and even more so in children under 3 years of age.

Therefore, the technical problem raised is to develop a natural liquid composition against infections that makes it possible to replace the 70° alcohol boricated to saturation with another product that is equally effective in the treatment of infections, but which eliminates the aforementioned problems related to both the final cost of the liquid composition and the storage difficulties.

### Description of the invention

The natural liquid composition against infections of this patent of addition presents constructive particularities aimed at solving the technical problem raised, by avoiding the use of 70° alcohol boricated to saturation, and achieving a liquid composition that improves its healing or curative properties and is more economical, about 9.8888... times, than that disclosed in patent of invention no. 200400524.

To this end, and according to the invention, this natural liquid composition against infections comprises between 0.5 and 1 millilitre of 70° ethyl alcohol for every 10 millilitres of an infusion of chamomile (Chamomilla recutita or Matricaria chamomilla), naturally grown, without chemical fertilisers or artificial pesticides; the infusion whereof is made with one gram of chamomile for every 0.2 litres of low-mineralisation mineral water, preferably from the Pyrenees.

The properties of this natural liquid composition make it especially indicated for the treatment of infections, and as a lotion for the treatment of muscle and joint pain in the human body.

The liquid composition of the present invention is applied directly on the infected area, by applying the product on the damaged area with cotton several times a day; in this way, the infection that may be present in the same area disappears 24 hours after applying the product. Moreover, this liquid composition is effective in healing mouth infections, and other infections in general, as well as against muscle and joint inflammation and pain, in this case by applying it as a lotion on the affected area.

The liquid obtained does not present any secondary or collateral effects, and, in and of itself, is an evident solution for any type of infection, since it provides users with a product designed for comprehensive treatment, with different uses, which include:
- the preparation of a natural medicament designed to treat ear disorders, such as chronic otitis, pain, inflammation, infections, palpitations, and perforation of the ear drum;
- the preparation of a natural medicament designed to treat eye disorders, such as irritation, rheum, styes;
- the preparation of a natural medicament designed to disinfect stings from insects, wasps, ticks, etc.;
- the preparation of a natural medicament designed to disinfect and decongest the respiratory tract;
- the preparation of a natural medicament designed to treat gum infections, inflammation and wounds, and throat infections;
- the preparation of a natural medicament designed to disinfect external wounds, skin irritation, herpes, itching sensations, stinging sensations, eczemas, fungi, blemishes, styes, acne and similar disorders;
- the preparation of a natural medicament designed to disinfect haemorrhoids;
- the use as an aftershave lotion for sensitive skins;
- the use as a lotion against muscle and joint inflammation and pain;
- the use as a lotion against the redness and pain in the big toes caused by uric acid, by applying the liquid composition directly on the user.

In a variant embodiment developed especially for use as a treatment against psoriasis, the liquid composition includes, in its composition, organic sweet almond oil in a quantity ranging between 3 and 6 millilitres for every 10 millilitres of chamomile infusion.

### Preferred embodiment of the invention

In a basic preferred embodiment for the uses mentioned above, the liquid composition of the invention comprises 0.625 millilitres of 70° ethyl alcohol for every 10 millilitres of organic chamomile infusion.

The organic chamomile infusion is prepared in a proportion of 1 gram of chamomile (Chamomilla recutita or Matricaria Chamomilla) for every 0.2 litres of low-mineralisation mineral water.

In a specific embodiment for the treatment of psoriasis, the liquid composition further includes in its composition organic sweet almond oil in a quantity of 5 millilitres for every 10 millilitres of chamomile infusion and 0.625 millilitres of 70° ethyl alcohol.

Prior to the topical application of this liquid composition in a treatment against psoriasis, it is advisable to previously wash the affected areas with bath gel for delicate, very dry skins, since this enhances the effectiveness of the anti-psoriasis treatment.

## Claims

1. Addition to patent of invention no. 200400524 for "Natural liquid composition against infections", **characterised in that** said liquid composition comprises between 0.5 and 1 millilitre of 70° ethyl alcohol for every 10 millilitres of an infusion of chamomile (Chamomilla recutita or Matricaria Chamomilla), naturally grown, without chemical fertilisers or pesticides, prepared with 1 gram of chamomile for every 0.2 litres of low-mineralisation mineral water.

2. Liquid composition, according to claim 1, **characterised in that** it uses, as the only active principle, chamomile in the form of an infusion dissolved in alcohol.

3. Liquid composition, according to claim 1, **characterised in that** its composition includes organic sweet almond oil in a quantity ranging between 3 and 6 millilitres for every 10 millilitres of chamomile infusion.

4. Use of the liquid composition according to claim 1, to prepare a natural medicament designed to treat ear disorders, such as chronic otitis, pain, inflammation, infections, palpitations, perforation of the ear drum.

5. Use of the liquid composition according to claim 1, to prepare a natural medicament designed to treat eye disorders, such as irritation, rheum, styes.

6. Use of the liquid composition according to claim 1, to prepare a medicament designed to disinfect stings from insects, wasps, mosquitoes, ticks, etc.

7. Use of the liquid composition according to claim 1, to prepare a natural medicament designed to disinfect and decongest the respiratory tract.

8. Use of the liquid composition according to claim 1, to prepare a natural medicament designed to treat gum inflammation and wounds, and throat infections.

9. Use of the liquid composition according to claim 1, to prepare a natural medicament designed to disinfect external wounds, skin irritation, herpes, itching sensations, stinging sensations, eczemas, fungi, blemishes, styes, acne, etc.

10. Use of the liquid composition according to claim 1, as an aftershave lotion for sensitive skins.

11. Use of the liquid composition according to claim 1, as a lotion against muscle and joint inflammation and pain in human beings.

12. Use of the liquid composition according to claim 1, as a lotion against the redness and pain in the big toes caused by uric acid.

13. Use of the liquid composition according to the preceding claims, wherein the composition is directly applied.

14. Use of the liquid composition according to claim 3, as a treatment against psoriasis.
